# EUROPEAN PATENT APPLICATION

(11) **EP 4 085 900 A1**
(43) Date of publication of application: **09.11.2022**
(21) Application number: 21751062.7
(22) Date of filing: 05.02.2021
(51) Int. Cl.: A61K 9/127, A61K 9/00, A61K 38/17, A61P 35/00

(54) **PULMONARY SURFACTANT PARTICLE LOADED WITH NUCLEIC ACID OR PROTEIN FOR INHALED DELIVERY, AND METHOD FOR PRODUCING SAME**

(30) Priority: 05.02.2020 KR 20200013867
(71) Applicant: Korea Advanced Institute of Science and Technology, Daejeon 34141 (KR)
(72) Inventor: PARK, Ji Ho, Daejeon 34141 (KR); OH, Chan Hee, Daejeon 34141 (KR)
(74) Representative: EP&C
(86) International application number: PCT/KR2021/001516
(87) International publication number: WO 2021/158053

(57) **Abstract**

A complex in which a protein or nucleic acid is bound to a liposome prepared with a pulmonary surfactant can be selectively delivered to the lung while preserving its original form and function without damaging the structure of the protein or nucleic acid when administered in vivo. In addition, the complex is efficiently fused with the pulmonary surfactant membrane present in the alveoli to efficiently deliver the bound protein or nucleic acid (gene) to surrounding cells, and has low toxicity and excellent structural stability.

## Description

### BACKGROUND OF THE INVENTION

### 1. Field of the Invention

The present invention relates to a method of optimizing a particle formation using biocompatible pulmonary surfactant particles and positively charged protamine protein to deliver negatively charged proteins or nucleic acids deep into the lungs. In order to load a negatively charged protein or nucleic acid, it was attempted to combine with particles by an attractive force through a positively charged protamine as a medium. The reason is that it is intended to be delivered in a state that preserves its original form without damaging the structure of the protein or nucleic acid.

### 2. Description of the Related Art

The number of patients with lung disease is increasing significantly every year, and among all patients with lung disease, patients with respiratory virus infection belong to the category with the highest mortality rate. Accordingly, there is a need for a delivery system that can selectively target the lung and deliver the drug or protein in a state that preserves the original form and function without damaging the structure.

In particular, among viruses that cause respiratory infections, influenza and coronavirus mainly infect alveolar type II cells in the alveolar region. Therefore, there is a need for a technique for efficiently delivering an immunopotentiator and an antiviral agent to the alveolar region, particularly to type II alveolar cells.

The alveolar type II cells function to secrete and store pulmonary surfactant, and the pulmonary surfactant plays a role in regulating lung tension during respiration. The pulmonary surfactant is composed of lipid and membrane protein (Non-patent reference, Eur Respir J. 1999 Jun; 13(6):1455-76).

If proteins or nucleic acids are delivered to the alveoli using the pulmonary surfactant, it was expected that proteins or nucleic acids could be maintained in the alveoli for a long time through fusion with the pulmonary surfactant membrane in the alveoli, and to be able to effectively target the proteins or nucleic acids to alveolar type II cells that secrete and store the pulmonary surfactant. The present invention was completed by preparing a complex in which a protein or nucleic acid is bound to a liposome prepared with a pulmonary surfactant.

### [PRIOR ART REFERENCE]

### [NON-PATENT REFERENCE]

(Non-Patent Reference 1) Eur Respir J. 1999 Jun; 13(6):1455-76

### SUMMARY OF THE INVENTION

It is an object of the present invention to provide a complex for delivery of a protein or nucleic acid for the selective delivery of a protein or nucleic acid to the lung.

It is another object of the present invention to provide a method for preparing a complex for delivery of a protein or nucleic acid for the selective delivery of a protein or nucleic acid to the lung.

To achieve the above objects, the present invention provides a complex for delivery of a protein or nucleic acid, wherein the complex is a protein or nucleic acid bound to a liposome prepared with a pulmonary surfactant.

The present invention also provides a method for preparing a complex for delivery of a protein or nucleic acid comprising the following steps:
preparing a first solution in which a pulmonary surfactant is dissolved;
forming a lipid film by drying the prepared solution in which the pulmonary surfactant is dissolved; and
preparing a complex in which a binding target protein or nucleic acid is bound to a liposomes prepared with a pulmonary surfactant by treating the formed lipid film with a second solution in which the binding target protein or nucleic acid is dissolved to hydrate.

### ADVANTAGEOUS EFFECT

A complex in which a protein or nucleic acid is bound to a liposome prepared with a pulmonary surfactant provided in one aspect of the present invention can be selectively delivered to the lung while preserving its original form and function without damaging the structure of the protein or nucleic acid when administered in vivo. In addition, the complex is efficiently fused with the pulmonary surfactant membrane present in the alveoli to efficiently deliver the bound protein or nucleic acid (gene) to surrounding cells, and has low toxicity and excellent structural stability.

### BRIEF DESCRIPTION OF THE DRAWINGS

Figure 1 is a diagram showing the schematic diagram of a protein loading method using a pulmonary surfactant.
Figure 2a is a diagram showing the results of measuring the green fluorescent protein (GFP) loading rate as increasing the amount of protamine in a molar ratio of 1:1, 1:5, 1:10, 1:20 based on 1 mol of the green fluorescent protein (GFP) in a state where the amounts of the green fluorescent protein (GFP) and pulmonary surfactant were fixed at 50 µg and 5 mg, respectively.
Figure 2b is a diagram showing the results of measuring the particle size as increasing the amount of protamine in a molar ratio of 1:1, 1:5, 1:10, 1:20 based on 1 mol of the green fluorescent protein (GFP) in a state where the amounts of the green fluorescent protein (GFP) and pulmonary surfactant were fixed at 50 µg and 5 mg, respectively.
Figure 2c is a diagram showing the results of measuring the charge as increasing the amount of protamine in a molar ratio of 1:1, 1:5, 1:10, 1:20 based on 1 mol of the green fluorescent protein (GFP) in a state where the amounts of the green fluorescent protein (GFP) and pulmonary surfactant were fixed at 50 µg and 5 mg, respectively.
Figure 3 is a diagram showing the photograph of the shape of the pulmonary surfactant particles loaded with the green fluorescent protein observed under an electron microscope (TEM).
Figure 4a is a diagram showing the results of confirming the changes in the particle size over time at 37°C after the particles were placed in a culture medium containing 10% (v/v) FBS (fetal bovine serum), which is an environment similar to that of a living body, in order to evaluate the stability of the completed particles.
Figure 4b is a diagram showing the results of confirming the changes in GFP fluorescence over time at 37°C after the particles were placed in a culture medium containing 10% (v/v) FBS (fetal bovine serum), which is an environment similar to that of a living body, in order to evaluate the stability of the completed particles.
Figure 4c is the results of confirming the particle toxicity.
Figure 5 is a diagram showing the results of confirming the intracellular delivery observed under a confocal microscope after treating a protamine-protein complex (Pro-GFP), the control group, to human alveolar epithelial cells (HPAepic), macrophages (Raw 264.7), or type II alveolar cell-derived lung cancer cells (A549) for 24 hours, respectively.
Figure 6 is a diagram showing the results of confirming the intracellular delivery observed under a confocal microscope after treating pulmonary surfactant-protein-protamine (Surf-Pro-GFP) particles to human alveolar epithelial cells (HPAepic) for 24 hours.
Figure 7 is a diagram showing the results of confirming the intracellular delivery observed under a confocal microscope after treating pulmonary surfactant-protein-protamine (Surf-Pro-GFP) particles to macrophages (Raw 264.7) for 24 hours.
Figure 8 is a diagram showing the results of confirming the intracellular delivery observed under a confocal microscope after treating pulmonary surfactant-protein-protamine (Surf-Pro-GFP) particles to type II alveolar cell-derived lung cancer cells (A549) for 24 hours.
Figure 9 is a diagram showing the results of comparing the case in which only the control GFP protein was delivered to the lungs by inhalation and the case in which pulmonary surfactant-protein-protamine (Surf-Pro-GFP) particles were delivered in order to confirm whether pulmonary surfactant-protein-protamine (Surf-Pro-GFP) particles were well delivered to the lungs in an actual small animal mouse model.
Figure 10 is a diagram showing the results of confirming the organ distribution of proteins and particles over time, after producing pulmonary surfactant-protein-protamine (Surf-Pro-GFP) particles based on 1 mg of the pulmonary surfactant and delivering the particles to a mouse animal model by inhalation, in order to confirm the organ distribution of the pulmonary surfactant-protein-protamine (Surf-Pro-GFP) particles over time.
Figure 11a is a diagram showing the graph in which the DiR fluorescence results of Figure 10, the results of confirming the organ distribution of proteins and particles over time, after producing pulmonary surfactant-protein-protamine (Surf-Pro-GFP) particles based on 1 mg of the pulmonary surfactant and delivering the particles to a mouse animal model by inhalation, are quantified.
Figure 11b is a diagram showing the graph in which the GFP fluorescence results of Figure 10, the results of confirming the organ distribution of proteins and particles over time, after producing pulmonary surfactant-protein-protamine (Surf-Pro-GFP) particles based on 1 mg of the pulmonary surfactant and delivering the particles to a mouse animal model by inhalation, are quantified.
Figure 12 is a diagram showing the schematic diagram of a nucleic acid loading method using a pulmonary surfactant.
Figure 13 is a diagram showing the results of measuring the size and charge of the siRNA-protamine complex using DLS equipment.
Figure 14 is a diagram showing the results of confirming the intracellular delivery observed under a confocal microscope after treating pulmonary surfactant-protamine-siRNA (Surf-Pro-siRNA) particles to type II alveolar cell-derived lung cancer cells (A549) for 24 hours.
Figure 15 is a diagram showing the results of confirming the luminescence through IVIS equipment after treating Luc-siRNA to A549 cells expressing luminescence (A549-luc) in order to confirm the protein expression reduction ability of Luc-siRNA using the pulmonary surfactant.
Figure 16a is a diagram showing the results of confirming the cell death rate through MTT technique after treating type II alveolar cell-derived lung cancer cells (A549-luc) with each experimental group.
Figure 16b is a diagram showing the quantitative results of confirming the luminescence through IVIS equipment to confirm the protein expression reduction ability of Luc-siRNA using the pulmonary surfactant.
Figure 17 is a diagram showing the results of confirming the apoptosis effect through MTT technique using KRAS-siRNA and RPN2-siRNA known as siRNAs capable of inducing apoptosis in type II alveolar cell-derived lung cancer cells (A549-luc) through the existing literature, and the pulmonary surfactant.

### DESCRIPTION OF THE PREFERRED EMBODIMENTS

Hereinafter, the present invention is described in detail.

The embodiments of this invention can be modified in various other forms, and the scope of the present invention is not limited to the embodiments described below. It is well understood by those in the art who has the average knowledge on this field that the embodiments of the present invention are given to explain the present invention more precisely.

In addition, the "inclusion" of an element throughout the specification does not exclude other elements, but may include other elements, unless specifically stated otherwise.

The present invention provides a complex for delivery of a protein or nucleic acid, wherein the complex is a protein or nucleic acid bound to a liposome prepared with a pulmonary surfactant.

The binding of the protein or nucleic acid to the liposome can be bound and/or encapsulated inside the liposome, or bound to the outside of the liposome.

The pulmonary surfactant can be a bio-derived pulmonary surfactant, and in the present invention, a complex for delivery of a protein or nucleic acid was prepared using a bio-derived pulmonary surfactant through Examples. In addition, the pulmonary surfactant is not limited to the bio-derived pulmonary surfactant, and can include a mimic bio-derived pulmonary surfactant, a synthetic pulmonary surfactant, and a semi-synthetic (ie, modified natural) pulmonary surfactant.

The surfactant that mimics the bio-derived pulmonary surfactant or the synthetic or semi-synthetic pulmonary surfactant has more than 10%, 20%, 30%, 40%, 50%, 60%, 70%, 80% and 90% protein homology with the bio-derived pulmonary surfactant.

In this case, the protein bound to the liposome is bound to the liposome through a positively charged protein or peptide. As the positively charged protein, protamine, histone, lysozyme, and the like can be used, and preferably, FDA-approved protamine can be used.

The positively charged protein or peptide is preferably a protein or peptide including a domain rich in cationic amino acids. The cationic amino acid includes arginine, histidine, and lysine. For example, the arginine-rich protein includes protamine.

Accordingly, the protein binding to the liposome is preferably a protein having a negative charge. A positively charged protamine is used as a linker to load a negatively charged protein, and the reason is to deliver the protein in a state that preserves the original protein form without damaging the protein structure.

The nucleic acid binding to the liposome can be, for example, DNA or RNA, and the RNA can be, for example, mRNA (messenger RNA), rRNA (ribosomal RNA), tRNA (transfer RNA), snRNA (small nuclear RNA), snoRNA (small nucleolar RNA), aRNA (antisense RNA), miRNA (micro RNA), siRNA (small interfering RNA), or piRNA (piwi interacing RNA).

In addition, the nucleic acid includes a gene therapy agent.

The complex for delivery of a protein or nucleic acid can selectively deliver the protein or nucleic acid bound to the liposome to the lung, which is due to the interaction between the pulmonary surfactant forming the liposome and the pulmonary surfactant membrane and cells present in the lung.

More specifically, the pulmonary surfactant can be a lipid-protein complex composed of the lipids and proteins produced in type II alveolar cells. That is, the pulmonary surfactant may include a mammalian pulmonary surfactant collected from the mammalian lung. In this case, the mammal can be a human, and can be an animal other than a human, specifically, a pig or a cow.

In general, since a natural pulmonary surfactant is secreted and stored in type II alveolar cells, the liposome based on the pulmonary surfactant can efficiently target type II alveolar cells and deliver the bound protein and nucleic acid.

The term lipid in the lipid-protein complex refers to a natural, synthetic or semi-synthetic (ie, modified natural) compound that is generally amphiphilic. Lipid typically includes a hydrophilic component and a hydrophobic component. Lipid includes phospholipid, fatty acid, fatty alcohol, triglyceride, phosphatide, oil, glycolipid, aliphatic alcohol, wax, terpene and steroid, but not always limited thereto. The "semi-synthetic (or modified natural) compound" refers to a natural compound that has been chemically modified in some way.

Examples of phospholipid include natural and/or synthetic phospholipids.

Usable phospholipid includes phosphatidylcholine (saturated and unsaturated), phosphatidylglycerol, phosphatidylethanolamine, phosphatidylserine, phosphatidic acid, phosphatidylinositol, sphingolipid, diacylglyceride, cardiolipin, ceramide and cerebroside, but not always limited thereto. For example, phospholipid includes dipalmitoyl phosphatidylcholine (DPPC), dilauryl phosphatidylcholine (DLPC) (C12:0), dimyristoyl phosphatidylcholine (DMPC) (C14:0), distearoyl phosphatidylcholine (DSPC), diphytanoyl phosphatidylcholine, nonadecanoyl phosphatidylcholine, arakidoyl phosphatidylcholine, dioleoyl phosphatidylcholine (DOPC) (C18:1), dipalmitoleoyl phosphatidylcholine (C16:1), linoleoyl phosphatidylcholine (C18:2), myristoyl palmitoyl phosphatidylcholine (MPPC), steroyl myristoyl phosphatidylcholine (SMPC), steroyl palmitoyl phosphatidylcholine (SPPC), palmitoyloleoyl phosphatidylcholine(POPC), palmitoyl palmitoleoyl phosphatidylcholine (PPoPC), dipalmitoyl phosphatidylethanolamine (DPPE), palmitoyloleoyl phosphatidylethanolamine (POPE), dioleoylphosphatidylethanolamine (DOPE), dimyristoylphosphatidylethanolamine (DMPE), distearoyl phosphatidylethanolamine (DSPE), dioleoyl phosphatidylglycerol (DOPG), palmitoyloleoyl phosphatidylglycerol (POPG), dipalmitoyl phosphatidylglycerol (DPPG), dimyristoyl phosphatidylglycerol (DMPG), distearoyl phosphatidylglycerol (DSPG), dimyristoylphosphatidylserine (DMPS), distearoylphosphatidylserine (DSPS), palmitoyloleoyl phosphatidylserine (POPS), soybean lecithin, egg yolk lecithin, sphingomyelin, phosphatidylinositol, diphosphatidylglycerol, phosphatidylethanolamine, phosphatidic acid and egg phosphatidylcholine (EPC), but not always limited thereto.

Examples of fatty acid and fatty alcohol include sterol, palmitic acid, cetyl alcohol, lauric acid, myristic acid, stearic acid, phytanic acid, dipalmitic acid, and the like, but not always limited thereto. Exemplary fatty acid includes palmitic acid.

Examples of fatty acid ester include methyl palmitate, ethyl palmitate, isopropyl palmitate, cholesteryl palmitate, palmityl palmitate, sodium palmitate, potassium palmitate and tripalmitine and the like, but not always limited thereto.

On the other hand, the pulmonary surfactant contains a membrane protein. The presence of this membrane protein makes it possible to selectively and effectively target type II alveolar cells. The membrane protein can include at least one natural surfactant polypeptide selected from the group consisting of SP-A, SP-B, SP-C, and SP-D, a part or a mixture thereof. Exemplary peptide can contain at least about 1 to 50 amino acids of a natural surfactant polypeptide, or 5, 10, 15, 20, 25, 30, 35, 40, 45 or 50 amino acid fragments. Exemplary SP-B polypeptide can contain at least about 1 to 50 amino acids of SP-B, or 5, 10, 15, 20, 25, 30, 35, 40, 45 or 50 amino acid fragments. SP-B peptide can be an amino-terminal peptide or a carboxy-terminal peptide. Exemplary SP-B peptide can be an amino-terminal peptide of 25-amino acids.

In another embodiment of the present invention, the pulmonary surfactant can include a surfactant polypeptide generated recombinantly. Recombinant SP-A, SPB, SP-C, SP-D or a portion thereof can be obtained by expressing a DNA sequence encoding the SP-A, SP-B, SP-C, SP-D or the portion thereof in a suitable prokaryotic or eukaryotic expression system using various known techniques. Recombinant vectors readily adapted to contain the isolated nucleic acids encoding a surfactant polypeptide or a portion thereof, host cells containing the recombinant vectors and methods of preparing such vectors and host cells, as well as their use in the production of the encoded polypeptides by recombinant techniques are well informed. A nucleic acid sequence encoding a surfactant polypeptide or a portion thereof can be provided in an expression vector comprising a nucleotide sequence encoding a surfactant polypeptide operably linked to at least one regulatory sequence. It should be understood that the design of the expression vector may depend on the factors such as the type of a protein desired for selection and/or expression of the host cell to be transformed. The number of copies of the vector, the control ability of the copy number, and the expression of any other protein encoded by the vector (eg, antibiotic marker) should be considered. For example, a nucleic acid of the target can be used to produce proteins or polypeptides including fusion proteins or polypeptides, and to cause expression and excess expression of kinase and phosphatase polypeptide in the cells proliferated by culture.

In order to express a surfactant polypeptide or a portion thereof, host cells can be transfected with recombinant genes. The host cell can be any prokaryotic or eukaryotic cell. For example, polypeptides can be expressed in bacterial cells, for example E. coli, insect cells, yeast or mammalian cells. In these examples, if the host cell is a human cell, it may or may not be in a living object. Other suitable host cells are known to those skilled in the art. Additionally, the host cell can be supplemented with tRNA molecules not typically found in the host, in order to optimize the expression of a polypeptide. Other methods suitable for maximizing the expression of a polypeptide are known to those skilled in the art. Methods for preparing polypeptides are well known in the art. For example, host cells transfected with an expression vector encoding a surfactant polypeptide or a portion thereof can be cultured under appropriate conditions to allow the expression of the polypeptide. The polypeptide can be secreted and isolated from a mixture of cells and the medium containing the polypeptide. Alternatively, the polypeptide can be preserved cytoplasmically. Then, the cells are harvested, lysed, and proteins are isolated from cell lysates.

The surfactant polypeptide and surfactant lipid are interacted by hydrostatic interaction. The charged amino acids interact with the polar head group of lipids, and the hydrophobic amino acids interact with the phospholipid acyl side chain. For example, SP-B and SP-C are hydrophobic proteins. Both SP-B and SP-C bind preferentially to anionic lipids (eg, phosphatidylglycerol (PG)). SP-A and SP-D are hydrophilic proteins and interact with a wide range of amphiphilic lipids, including glycerophospholipids, sphingophospholipids, sphingoglycolipids, lipid A and lipoglycans. SP-A binds to DPPC. As an example, hydrostatic interaction of KL4, a SP-B mimetic, with lipids in natural surfactants or lipids contained in surface activators is observed. For example, a lysine residue in the KL4 peptide interacts with the charged head group of DPPC, and a hydrophobic leucine residue interacts with the phospholipid acyl side chain of phosphatidylglycerol.

On the other hand, the average diameter range of a complex in which a protein is bound to a liposome prepared with a pulmonary surfactant is not particularly limited. In some embodiments of the present invention, the average diameter range can be from 250 to 1200 nm, from 260 to 1200 nm, from 270 to 1200 nm, from 280 to 1200 nm, from 290 to 1200 nm, from 300 to 1200 nm, from 250 to 1150 nm, from 250 to 1120 nm, from 250 to 1110 nm, from 260 to 1150 nm, from 270 to 1120 nm, from 280 to 1110 nm, from 298 to 1108 nm, or from 298.9 to 1108 nm. Or, the average diameter range can be 1 µm or less, and if it is 1 µm or more, it may be difficult to deliver the liposome to the lung alveolus.

The average diameter of the complex can be appropriately controlled by adjusting the amount of the pulmonary surfactant, positively charged protein or peptide used in preparing the complex using an extruder kit.

The complex for delivery of a protein or nucleic acid can be delivered to the lungs by inhalation. Inhalation devices such as inhalers (including dry powder inhalers and metered dose inhalers) and nebulizers (also known as atomizers) can be used to deliver the bound protein or nucleic acid to the lungs.

An exemplary dry powder inhaler can be obtained from Inhale Therapeutic Systems. The dry powder inhaler can also be obtained from 3M.

In another aspect of the present invention, the present invention provides a method for preparing a complex for delivery of a protein or nucleic acid comprising the following steps:
preparing a first solution in which a pulmonary surfactant is dissolved (step 1);
forming a lipid film by drying the prepared solution in which the pulmonary surfactant is dissolved (step 2); and
preparing a complex in which a binding target protein or nucleic acid is bound to a liposome prepared with a pulmonary surfactant by treating the formed lipid film with a second solution in which the binding target protein or nucleic acid is dissolved (step 3).

Hereinafter, the method for preparing a complex for delivery of a protein or nucleic acid provided in one aspect of the present invention is described in detail step by step.

In the method for preparing a complex for delivery of a protein or nucleic acid provided in one aspect of the present invention, step 1 is a step of preparing a first solution in which the pulmonary surfactant to form liposomes is dissolved.

As the type of a solvent used for dissolving the pulmonary surfactant, any solvent that can easily dissolve the pulmonary surfactant can be used without limitation, and in one embodiment, chloroform, methanol, etc. can be used alone or in combination.

Due to the unique characteristics of a pulmonary surfactant, liposomes prepared with the pulmonary surfactant have the effect of selectively targeting the lungs by interaction with cells existing in the lungs.

Meanwhile, the pulmonary surfactant is a complex composed of lipids and proteins produced in type II alveolar cells (i.e, lipid-protein complex), and the pulmonary surfactant preferably contains a membrane protein.

In one aspect, the complex in which a protein or nucleic acid is bound to the liposome prepared with the pulmonary surfactant can target type II alveolar cells.

However, this is not intended to limit the delivery of the liposome prepared with the pulmonary surfactant according to the present invention to type II alveolar cells or lung cancer cells derived from type II alveolar cells, but only an example of pulmonary delivery.

In the method for preparing a complex for delivery of a protein or nucleic acid provided in one aspect of the present invention, step 2 is a step of forming a lipid film by drying the prepared solution in which the pulmonary surfactant is dissolved.

The method for forming the lipid film can be carried out by the conventional known method, and in one embodiment, a solution in which the pulmonary surfactant is dissolved is placed in a glass vial and dried at room temperature to form a lipid film.

In the method for preparing a complex for delivery of a protein or nucleic acid provided in one aspect of the present invention, step 3 is a step of preparing a complex in which a binding target protein or nucleic acid is bound to a liposome prepared with a pulmonary surfactant by treating the formed lipid film with a second solution in which the binding target protein or nucleic acid is dissolved

At this time, in the step of hydrating the formed lipid film by treating the second solution in which the binding target protein or nucleic acid is dissolved, the third solution in which the positively charged protein or peptide is dissolved is also treated to induce binding of the protein or nucleic acid to the liposome through the positively charged protein or peptide.

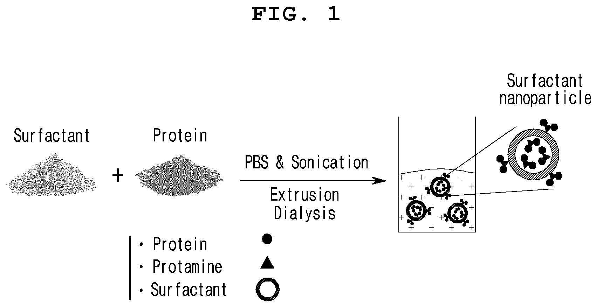

As mentioned above, most proteins or nucleic acids are negatively charged. Therefore, when the binding target protein or nucleic acid has a negative charge, a third solution in which the positively charged protein or peptide is dissolved in the hydration step of the lipid film is treated together. In this way, via the positively charged protein or peptide, the binding target protein or nucleic acid can be stably bound to the inside and/or outside of the liposome prepared with the pulmonary surfactant (Figure 1).

When the binding target protein or nucleic acid is stably bound inside and/or outside the liposome prepared with the pulmonary surfactant, it can be delivered to the lungs in a state in which the original protein or nucleic acid form is preserved without damage to the protein or nucleic acid structure when administered in vivo.

One aspect of the present invention is an invention for optimizing a particle-forming method using biocompatible pulmonary surfactant particles and positively charged protamine protein to deliver negatively charged proteins or nucleic acids deep into the lungs. This optimization can be achieved by specifying the amount of the pulmonary surfactant, binding target protein or nucleic acid, and positively charged protein or peptide as follows when performing the method for preparing a complex for delivery of a protein or nucleic acid.

More specifically, when performing the method for preparing a complex for delivery of a protein or nucleic acid, 100 to 140 weight part of the pulmonary surfactant is used based on 1 weight part of the binding target protein or nucleic acid.

At the same time, it is preferable to use 1 to 10 mol of the positively charged protein or peptide based on 1 mol of the binding target protein or nucleic acid.

In addition, it is preferable to use 0.5 to 20 weight part of the positively charged protein or peptide based on 1 weight part of the binding target nucleic acid.

(Weight of binding target nucleic acid)/(weight of positively charged protein or peptide) can be, for example, 0.5, 0.75, 1, 1.25, 1.5, 2, 4, and preferably 1.

In addition, the mass ratio of the pulmonary surfactant to the weight of the complex of the binding target nucleic acid and the positively charged protein or peptide can be 1:1 to 1:20, for example, 1:1 to 1:18, 1:2 to 1: 15, 1:2 to 1:13, 1:2 to 1:8, 1:8 to 1:10, or1:5 to 1:15.

In the case of the pulmonary surfactant, 100 to 130 weight part of the pulmonary surfactant can be used based on 1 weight part of the binding target protein or nucleic acid.

Preferably, 100 to 120 weight part of the pulmonary surfactant can be used based on 1 weight part of the binding target protein or nucleic acid.

More preferably, 100 to 110 weight part of the pulmonary surfactant can be used based on 1 weight part of the binding target protein or nucleic acid.

Most preferably, 100 weight part of the pulmonary surfactant can be used based on 1 weight part of the binding target protein or nucleic acid.

At the same time, in the case of the positively charged protein or peptide, 2 to 10 mol of the positively charged protein or peptide can be used based on 1 mol of the binding target protein or nucleic acid.

Preferably, 4 to 10 mol of the positively charged protein or peptide can be used based on 1 mol of the binding target protein or nucleic acid.

More preferably, 6 to 10 mol of the positively charged protein or peptide can be used based on 1 mol of the binding target protein or nucleic acid.

More preferably, 8 to 10 mol of the positively charged protein or peptide can be used based on 1 mol of the binding target protein or nucleic acid.

Most preferably, 10 mol of the positively charged protein or peptide can be used based on 1 mol of the binding target protein or nucleic acid.

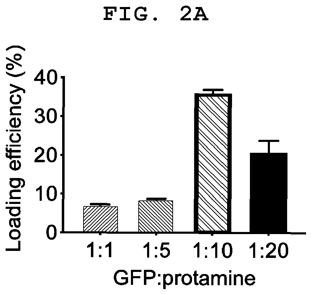

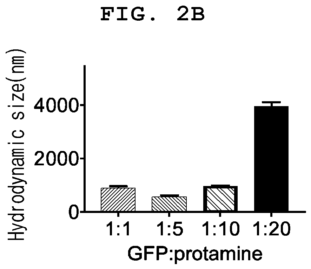

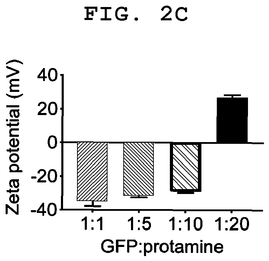

In one embodiment of the present invention, when the amount of the binding target protein or nucleic acid is fixed at 50 µg and the amount of the pulmonary surfactant is fixed at 5 mg, the results of the binding efficiency (%) of the binding target protein or nucleic acid that change as the amount of the positively charged protein or peptide is varied based on 1 mol of the binding target protein or nucleic acid are demonstrated in Figure 2. In the graph of Figure 2, the ratio on the x-axis means the used mol of the positively charged protein or peptide based on 1 mol of the binding target protein or nucleic acid.

When 1 mol, 5 mol, 10 mol, and 20 mol of the positively charged protein or peptide was used based on 1 mol of the binding target protein, the binding efficiency (%) of the binding target protein was gradually increased, but when the amount exceeded 10 mol, the binding efficiency (%) of the binding target protein was decreased significantly. In addition, when the amount of the positively charged protein or peptide exceeded 10 mol, the particle size showed a tendency to agglomerate abnormally, and the charge of the particle fluctuated and showed an unstable shape.

That is, from the results of Figure 2, it was confirmed that the excellent binding efficiency (%) of the binding target protein or nucleic acid was achieved only when 1 to 10 mol, particularly 10 mol of the positively charged protein or peptide based on 1 mol of the binding target protein or nucleic acid were used.

In summary, when performing the method for preparing a complex for delivery of a protein or nucleic acid, it is most preferable to use 100 weight part of the pulmonary surfactant based on 1 weight part of the binding target protein or nucleic acid and 10 mol of positively charged protein or peptide based on 1 mol of the binding target protein or nucleic acid at the same time.

On the other hand, the hydration step can be performed without limitation as long as the lipid film can be easily hydrated. For example, the hydration can be performed at a temperature range of 30 to 90°C, 35 to 90°C, 40 to 90°C, 45 to 90°C, 30 to 80°C, 30 to 70°C, 30 to 60°C, 30 to 50°C, 30 to 45°C, 35 to 55°C, 40 to 50°C, or at a temperature of 45°C.

In addition, a step of adjusting the diameter of the complex can be further included after performing the hydration step. In one embodiment of the present invention, the diameter of the composite was adjusted using an extruder kit, but it is not particularly limited thereto.

The average diameter range of a complex in which a protein or nucleic acid is bound to a liposome prepared with a pulmonary surfactant is not particularly limited. In some embodiments of the present invention, the average diameter range can be from 250 to 1200 nm, from 260 to 1200 nm, from 270 to 1200 nm, from 280 to 1200 nm, from 290 to 1200 nm, from 300 to 1200 nm, from 250 to 1150 nm, from 250 to 1120 nm, from 250 to 1110 nm, from 260 to 1150 nm, from 270 to 1120 nm, from 280 to 1110 nm, from 298 to 1108 nm, or from 298.9 to 1108 nm. Or, the average diameter range can be 1 µm or less, and if it is 1 µm or more, it may be difficult to deliver the liposome to the lung alveolus.

A complex in which a protein or nucleic acid is bound to a liposome prepared with a pulmonary surfactant provided in one aspect of the present invention can be selectively delivered to the lung while preserving its original form and function without damaging the structure of the protein or nucleic acid when administered in vivo. In addition, the complex is efficiently fused with the pulmonary surfactant membrane present in the alveoli to efficiently deliver the bound protein or nucleic acid (gene) to surrounding cells, and has low toxicity and excellent structural stability. These are supported by the Examples and Experimental Examples to be described later.

Hereinafter, the present invention will be described in detail by the following examples and experimental examples.

However, the following examples and experimental examples are only for illustrating the present invention, and the contents of the present invention are not limited thereto.

### Example 1: Preparation of protein-loaded pulmonary surfactant-based particles

Since the pulmonary surfactant has a strong negative charge and most proteins have a weak negative charge, a positively charged protamine protein was used as an intermediate linker to induce binding between the protein and the pulmonary surfactant.

The specific experimental procedure is as follows.
(1) A pulmonary surfactant solution was prepared by dissolving pulmonary surfactant powders (manufacturer: Yuhan Corporation / product name: NEWFACTEN) at a concentration of 10 mg/ml in a mixed solution of chloroform and methanol (2:1, v:v).
(2) A protamine protein solution was prepared by dissolving protamine in distilled water at a concentration of 2 mg/ml.
(3) A green fluorescent protein solution was prepared by dissolving green fluorescent protein (GFP) in distilled water at a concentration of 0.5 mg/ml.
(4) The pulmonary surfactant solution prepared in (1) was put in a glass vial, and the solvent was evaporated to form a lipid film.
(5) The protamine protein solution prepared in (2) and the green fluorescent protein solution prepared in (3) were put in the glass vial in which the pulmonary surfactant lipid film was formed, and the mixture was mixed on a hot plate at a temperature of 45°C to hydrate the pulmonary surfactant lipid film. The particles produced during the hydration process were made to have an average diameter of 400 nm using an extruder kit. In this process, the protamine protein and green fluorescent protein were bound to the inside and/or outside of the pulmonary surfactant-based particles.
(6) Thereafter, unbound proteins in the particles were removed through dialysis using a 100 kDa membrane.

A schematic diagram of a protein loading method using a pulmonary surfactant is shown in Figure 1.

In <Example 1>, green fluorescent protein (GFP) was used as a binding target protein or nucleic acid. The reason is that the green fluorescent protein is fluorescing, so it is advantageous to confirm quantitatively, and like most proteins, it has a weak negative charge.

Green fluorescent protein has a molecular weight of about 26.9 kDa, and has a negative charge of about -5 to -7 mV when the charge is measured through DLS (Dynamic light scattering) equipment. The corresponding contents are shown in Table 1 below.

**[Table 1]**

| | GFP |
|---|---|
| Molecular weight (kDa) | 26.9 |
| Zeta potential (mV) | -5 to -7 |

On the other hand, in order to achieve the maximum binding efficiency (%) of green fluorescent protein (GFP), a binding target protein or nucleic acid, pulmonary surfactant-based particles bound to proteins were prepared through the above-described process while variously controlling the amount of the pulmonary surfactant and protamine.

More specifically, for ratio optimization, the amounts of the green fluorescent protein (GFP) and pulmonary surfactant used in particle preparation were fixed at 50 µg and 5 mg, and the amount of protamine was varied. In a state where the amount of green fluorescent protein (GFP), a model protein, was fixed at 50 µg, the amount of pulmonary surfactant was fixed at 5 mg, and the amount of protamine was used from 1 (1:1 mol/mol) to 20 (1:20 mol/mol) times compared to the mol of the green fluorescent protein (GFP).

In addition, in order to observe intracellular uptake, the pulmonary surfactant was additionally labeled with DiI, a very hydrophobic and poorly watersoluble dye, as follows.

More specifically, a solution in which the red dye DiI was dissolved in methanol at a concentration of 1.25 mg/ml was separately prepared. The pulmonary surfactant solution prepared in (1) and the solution in which DiI is dissolved were mixed, and the pulmonary surfactant and DiI were adjusted so that they could be mixed in a ratio of 1000:1 by mass. The mixed solution was put in a glass vial, and the solvent was evaporated to form a lipid film. Subsequent processes were performed in the same manner as the above-described processes (5) and (6).

### Experimental Example 1: Evaluation of changes in loading rate (%) of green fluorescent protein (GFP), a binding target protein or nucleic acid, according to amount of pulmonary surfactant and protamine

Through comparison of the amount of the bound protein in the particles through green fluorescent protein (GFP) fluorescence before and after removing the unbound protein in the particles by dialysis in <Example 1>, the loading rate (%) change was evaluated. As described above, the amount of green fluorescent protein (GFP) was fixed at 50 µg.

In a state where the amounts of the green fluorescent protein (GFP) and pulmonary surfactant were fixed at 50 µg and 5 mg, respectively, the green fluorescent protein (GFP) loading rate, particle size and charge were measured with DLS as increasing the amount of protamine in a molar ratio of 1:1, 1:5, 1:10, 1:20 based on 1 mol of the green fluorescent protein (GFP).

The results are shown in Figure 2.

Figure 2 is a diagram showing the results of measuring the green fluorescent protein (GFP) loading rate, particle size and charge as increasing the amount of protamine in a molar ratio of 1:1, 1:5, 1:10, 1:20 based on 1 mol of the green fluorescent protein (GFP) in a state where the amounts of the green fluorescent protein (GFP) and pulmonary surfactant were fixed at 50 µg and 5 mg, respectively.

As shown in Figure 2, when the molar ratio was increased to more than 1:10, not only the loading rate was decreased, but also the particle size showed a tendency to agglomerate abnormally, and the charge of the particle fluctuated and showed an unstable shape. Therefore, it was finally decided to use 10 mol of protamine based on 1 mol of the green fluorescent protein (GFP).

In summary, it was confirmed that it is preferable to prepare the pulmonary surfactant-based particles to which the protein of <Example 1> is bound by using 100 weight part of the pulmonary surfactant based on 1 weight part of the binding target protein or nucleic acid (GFP) and 1 to 10 mol of protamine, particularly 10 mol based on 1 mol of the binding target protein (GFP).

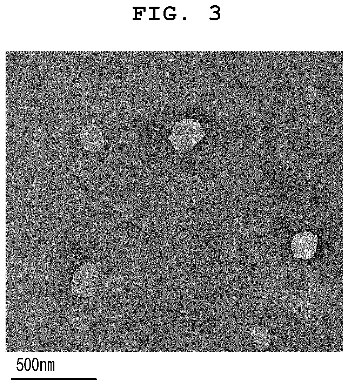

As shown in Figure 3, the shape of the pulmonary surfactant particles loaded with the green fluorescent protein was observed through the transmission electron micrographs of the particles. Through this, it was confirmed that the circular and uniform nanoparticles were well manufactured.

The particles prepared by using 100 weight part of the pulmonary surfactant based on 1 weight part of the binding target protein or nucleic acid (GFP) and 10 mol of protamine based on 1 mol of the binding target protein were used as the protein-loaded pulmonary surfactant-based particles in the following experimental examples.

### Experimental Example 2: Evaluation of particle stability

To evaluate the stability of the completed particles, the particles were put into a culture medium containing 10% FBS (fetal bovine serum), an environment similar to a living body, and the size of the particles was compared over time at 37°C. In addition, the stability of the particles was tested by comparing the particle sizes before and after the nebulizer treatment. In addition, the particle toxicity against A549 lung cancer cells derived from type II alveolar cells was tested.

**[Table 2]**

| | Particle size (nm) |
|---|---|
| Before nebulizer | 260.9 ± 4.88 |
| After nebulizer | 253.8 ± 12.93 |

As shown in table 2, the stability of the particles was confirmed by comparing the particle sizes before and after the nebulizer treatment. The stability of the particles was verified by confirming that the particles having a size of about 261 nm were formed before the nebulizer treatment, and the particles having a size of about 253.8 nm after the nebulizer treatment were formed.

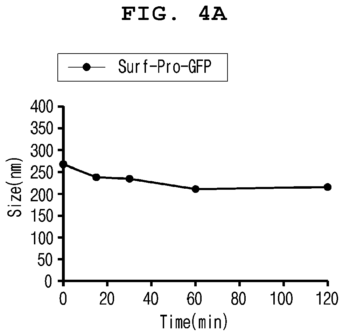

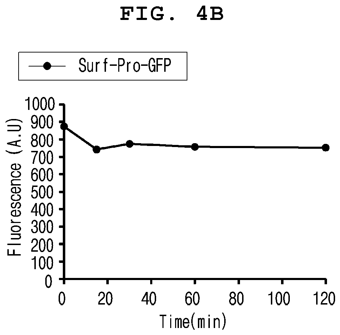

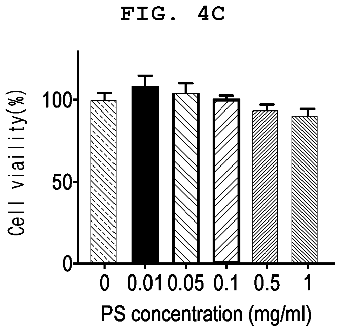

The results are shown in Figure 4.

Figure 4 is a diagram showing the results of confirming the changes in the particle size over time at 37°C after the particles were placed in a culture medium containing 10% (v/v) FBS (fetal bovine serum), which is an environment similar to that of a living body, in order to evaluate the stability of the completed particles.

As shown in Figure 4, when the size of the particles was examined with DLS equipment for each hour up to 120 minutes, the size did not change, confirming that the particles were stable in the in vivo environment. In addition, by confirming that the fluorescence of GFP did not change, it was confirmed that the loaded protein was not denatured in the corresponding environment.

In addition, in order to evaluate the toxicity of the particles themselves, A549, a type II alveolar cell-derived lung cancer cell line, was distributed in a 96-well plate (5000 cells/well), and one day later, the particles were treated thereto at the concentrations of 0.01, 0.05, 0.1, 0.5, and 1 mg/ml based on the pulmonary surfactant. After about 1 hour, the medium was replaced with PBS, and the cell death rate was confirmed 24 hours later. As a result, even when treated at the concentration of 1 mg/ml, the particle toxicity could not be observed.

### Experimental Example 3: Evaluation of intracellular delivery efficiency of pulmonary surfactant particles according to cell types

The intracellular delivery of the pulmonary surfactant-protein-protamine (Surf-Pro-GFP) particles was confirmed.

First, in order to verify the intracellular delivery effect through the pulmonary surfactant, a protamine-protein complex (Pro-GFP) without the pulmonary surfactant was used as a control to determine whether the intracellular delivery was achieved. Based on 7 µg of the protein (GFP), protamine was mixed in a molar ratio of 1:10 to form a complex. The complex had an average diameter of about 1364 nm and a positive charge of 15.3 mV. The prepared control complex was treated to human alveolar epithelial cells (HPAepic), macrophages (Raw 264.7), or type II alveolar cell-derived lung cancer cells (A549) for 24 hours, respectively, and the intracellular delivery was confirmed through a confocal microscope. At this time, the cells were subcultured in a 6-well plate (30,000 cells/well) 24 hours before the experiment.

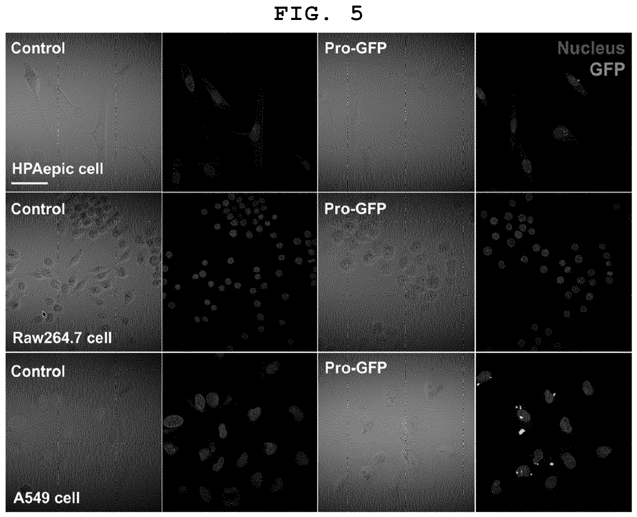

The results are shown in Figure 5.

Figure 5 is a diagram showing the results of confirming the intracellular delivery observed under a confocal microscope after treating a protamine-protein complex (Pro-GFP), the control group, to human alveolar epithelial cells (HPAepic), macrophages (Raw 264.7), or type II alveolar cell-derived lung cancer cells (A549) for 24 hours, respectively.

As shown in Figure 5, when the nucleus was stained with Hoechst dye for 5 minutes (blue) and the protein fluorescence (green) was observed under a confocal microscope, it was confirmed that the intracellular delivery did not occur smoothly in any of the three cell groups.

Next, pulmonary surfactant-protein-protamine (Surf-Pro-GFP) particles were treated to the same cells. 0.2 mg of pulmonary surfactant particles were labeled with DiI fluorescence (1.25 mg/ml in methanol) to confirm whether the particles (red) and protein (green) were well delivered into the cells. After the treatment for 24 hours, the cell nuclei were stained with Hoechst and the intracellular delivery was confirmed under a confocal microscopy. At this time, the cells were subcultured in a 6-well plate (30,000 cells/well) 24 hours before the experiment.

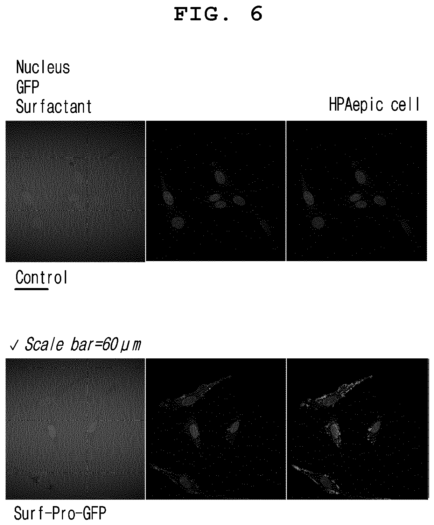

The results are shown in Figures 6, 7 and 8.

Figure 6 is a diagram showing the results of confirming the intracellular delivery observed under a confocal microscope after treating pulmonary surfactant-protein-protamine (Surf-Pro-GFP) particles to human alveolar epithelial cells (HPAepic) for 24 hours.

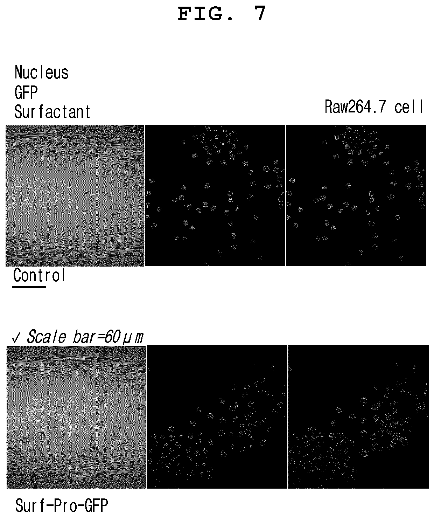

Figure 7 is a diagram showing the results of confirming the intracellular delivery observed under a confocal microscope after treating pulmonary surfactant-protein-protamine (Surf-Pro-GFP) particles to macrophages (Raw 264.7) for 24 hours.

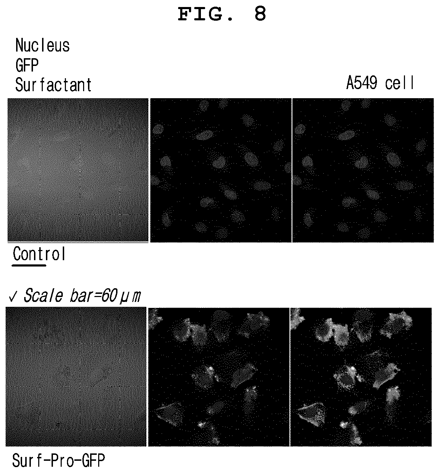

Figure 8 is a diagram showing the results of confirming the intracellular delivery observed under a confocal microscope after treating pulmonary surfactant-protein-protamine (Surf-Pro-GFP) particles to type II alveolar cell-derived lung cancer cells (A549) for 24 hours.

As shown in Figures 6, 7 and 8, it was confirmed that the Surf-Pro-GFP particles were hardly delivered into human alveolar epithelial cells (HPAepic cells). It was also confirmed that the Surf-Pro-GFP particles were hardly delivered into macrophages (Raw264.7). On the other hand, in the case of type II alveolar cell-derived lung cancer cells (A549), it was found that the particles were well delivered into the cells by interaction with the pulmonary surfactant, and the protein loaded on the particles was also well delivered into the cells.

### Experimental Example 4: Evaluation of delivery efficiency of pulmonary surfactant particles in animal mouse model

In order to confirm whether the SGP particles were well delivered to the lungs in an actual small animal mouse model, the case in which only the control GFP protein was delivered to the lungs by inhalation and the case in which the SGP particles were delivered were compared. At this time, the protein or SGP particles were delivered to the mouse through inhalation using a nebulizer of SCIREQ. 15 µg of protein and the same amount of protein-bound SGP particles were delivered to the animal model by inhalation. One hour later, in order to confirm whether the Surf-Pro-GFP particles were well delivered to the lungs in an actual small animal mouse model, the case in which only the control GFP protein was delivered to the lungs by inhalation and the case in which the Surf-Pro-GFP particles were delivered were compared. At this time, the protein or SGP particles were delivered to the mouse through inhalation using a nebulizer of SCIREQ. After 15 µg of protein and the same amount of protein-bound SGP particles were delivered to the animal model by inhalation, the lungs were excised 1 hour later and the GFP signal in the lungs was analyzed using IVIS equipment.

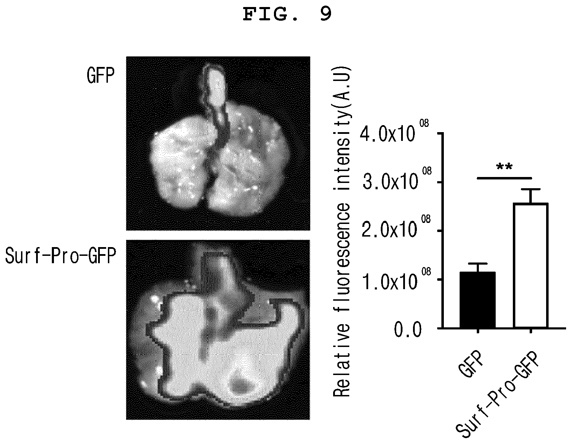

The results are shown in Figure 9.

Figure 9 is a diagram showing the results of comparing the case in which only the control GFP protein was delivered to the lungs by inhalation and the case in which the Surf-Pro-GFP particles were delivered in order to confirm whether the Surf-Pro-GFP particles were well delivered to the lungs in an actual small animal mouse model.

As shown in Figure 9, when only the GFP protein was delivered, the GFP protein did not spread throughout the lung and was concentrated in the airway, whereas when delivered as the Surf-Pro-GFP particles of <Example 1>, the GFP protein was delivered throughout the lung.

### Experimental Example 5: Evaluation of organ distribution of pulmonary surfactant particles over time after inhalation in animal mouse model

To confirm the organ distribution of Surf-Pro-GFP particles by time, Surf-Pro-GFP particles were prepared based on 1 mg of the pulmonary surfactant, and then delivered by inhalation to a mouse animal model, and the organ distribution of proteins and particles according to time was checked. At this time, the particles were labeled with DiI (1 mg/ml in methanol) fluorescence and the protein was observed through GFP fluorescence. After the inhalation delivery, the mice were incubated for 0 h, 1 h, 6 h, and 24 h, and the organs were extracted. Then, the distribution of the particles was confirmed. The distribution of DiR in the organs was confirmed using LiCoR equipment. In addition, the GFP signal was measured using IVIS equipment in the extracted organs.

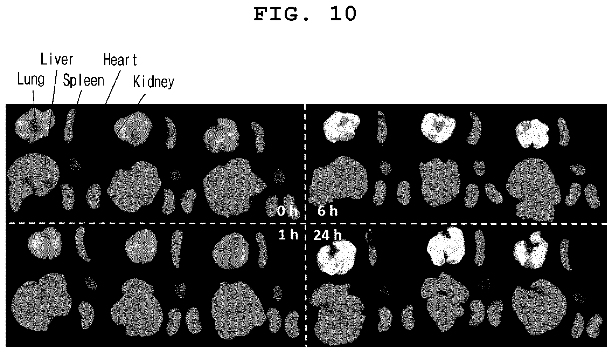

The results are shown in Figure 10.

Figure 10 is a diagram showing the results of confirming the organ distribution of proteins and particles over time, after producing Surf-Pro-GFP particles based on 1 mg of the pulmonary surfactant and delivering the particles to a mouse animal model by inhalation, in order to confirm the organ distribution of the Surf-Pro-GFP particles over time.

As shown in Figure 10, when checking the distribution of DiR in the organs, it was confirmed that the particles were distributed only in the lungs up to 24 hours. In Figure 10, red indicates organ autofluorescence, and green indicates pulmonary surfactant particles.

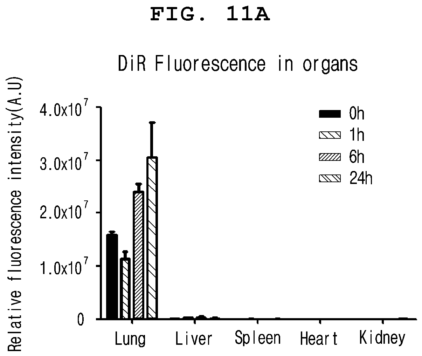

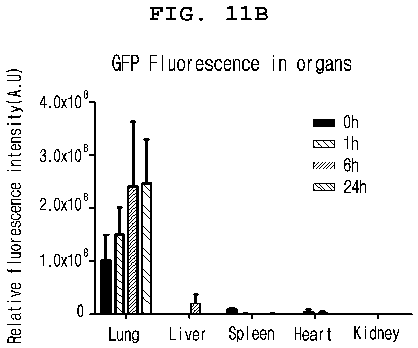

The results of quantifying the above results are shown in Figure 11.

Figure 11 is a diagram showing the graph in which the results of Figure 10, the results of confirming the organ distribution of proteins and particles over time, after producing pulmonary surfactant-protein-protamine (Surf-Pro-GFP) particles based on 1 mg of the pulmonary surfactant and delivering the particles to a mouse animal model by inhalation, are quantified.

As shown in Figure 11, both the particles and proteins were only in the lungs for 24 hours, and the trends of the particle and protein signals were similar. Therefore, it was confirmed that the particles and proteins delivered by inhalation remained well in the lungs and did not spread to other organs.

### Example 2: Preparation of nucleic acid-loaded pulmonary surfactant-based particles

### <2-1> Preparation of protamine-siRNA complex

To prepare nucleic acid-loaded pulmonary surfactant-based particles, a protamine-siRNA (Pro-siRNA) complex was prepared as follows. First, a siRNA solution was prepared by dissolving siRNA in distilled water at a concentration of 10 nmol/mL, and a protamine protein solution was prepared by dissolving protamine in distilled water at a concentration of 1 mg/mL. At this time, in order to measure the size and charge according to the mass ratio of siRNA and protamine, siRNA and protamine were mixed at the mass ratio of 0.5:1, 0.75:1, 1:1, 1.25:1, 1.5:1, 2:1, 4:1 based on 2 nmol siRNA, and the mixture was reacted at room temperature for about 15 minutes to prepare a siRNA-protamine complex.

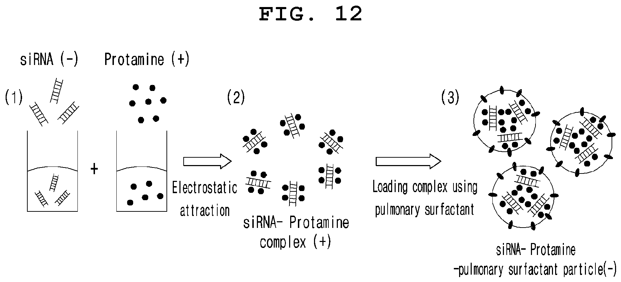

Figure 12 shows a schematic diagram of a nucleic acid loading method using a pulmonary surfactant.

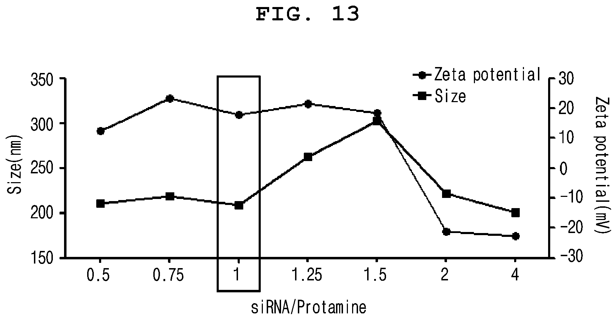

The size and charge of the prepared protamine-siRNA complex were measured using DLS equipment, and the results are shown in Figure 13. As a result, it was confirmed that as the mass ratio of siRNA to protamine increased, the protamine-siRNA complex became negatively charged. On the other hand, it is efficient to select a positively charged complex to be well loaded in the negatively charged pulmonary surfactant. Therefore, as can be seen in Figure 13, nucleic acid-loaded pulmonary surfactant-based particles were prepared by selecting a complex having a size of about 200 nm, a positive charge of 20 mV and a mass ratio of 1:1.

### <2-2> Preparation of siRNA-bound pulmonary surfactant-based particles

To prepare siRNA-bound pulmonary surfactant-based particles using the protamine-siRNA complex prepared in Example <2-1>, a pulmonary surfactant solution was prepared by dissolving the pulmonary surfactant (Newfacten) in a solution of chloroform and methanol (2:1) at a concentration of 10 mg/mL. Then, the pulmonary surfactant solution in a mass ratio of 1:5 or 1:10 to the protamine-siRNA complex prepared in Example <2-1> was placed in a glass vial, and the solvent was evaporated to form a lipid film. Thereafter, the siRNA-protamine complex was added to the lipid film and the hydration process was performed at about 45°C or higher to prepare siRNA-bound pulmonary surfactant-based particles.

The results of measuring the size and charge of the prepared siRNA-bound pulmonary surfactant-based particles using DLS equipment are shown in Table 3 below.

**[Table 3]**

| | Size (nm) | Surface charge (mV) |
|---|---|---|
| Pulmonary surfactant | | -40 |
| Protamine/siRNA complex (1:1 w/w) | 230 | +20 |
| Complex+pulmonary surfactant (1:5 w/w) | 240 | -25 |
| Complex+pulmonary surfactant (1:10 w/w) | 250 | -35 |

As shown in Table 3, the charge of the pulmonary surfactant alone was about -40 mV, and when the complex was well loaded in the pulmonary surfactant, the final particles also had a similar charge. When the ratio of the pulmonary surfactant to the complex was varied, it was confirmed that the particles of about 250 nm in size and -35 mV were produced when the particles were produced at a ratio of 1:10.

### Experimental Example 6: Evaluation of delivery efficiency of pulmonary surfactant particles into type II alveolar cell-derived lung cancer cells

Delivery of the pulmonary surfactant-protamine-siRNA (Surf-Pro-siRNA) particles prepared in Example 2 into type II alveolar cell-derived lung cancer cells was confirmed. In order to verify the effect of delivery into lung cells through the pulmonary surfactant, the protamine-siRNA (Pro-siRNA) without pulmonary surfactant prepared in Example <2-1> was used as the control to confirm the intracellular delivery.

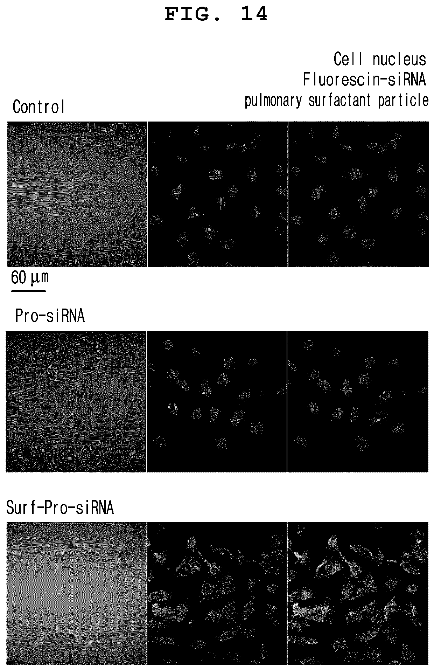

First, A549 cells were cultured in a 6-well plate (20000 cells/well), and one day later, the cells were treated with a negative control, protamine-Fluorescein-siRNA complex, and pulmonary surfactant-protamine-Fluorescein-siRNA (Surf-Pro-siRNA). At this time, the concentration of siRNA treated to all groups was fixed at 2 nmol/mL. After culturing for about 24 hours, the cells were photographed under a confocal microscope, and the results are shown in Figure 14.

As shown in Figure 14, it was confirmed that when only Pro-siRNA was used, it was not effectively delivered into type II alveolar cell-derived A549 lung cancer cells. On the other hand, it was confirmed that when siRNA was delivered using the pulmonary surfactant, it could be effectively delivered into type II alveolar cell-derived lung cancer cells.

### Experimental Example 7: Confirmation of protein expression reduction ability of siRNA using pulmonary surfactant

In order to confirm the protein expression inhibitory effect of siRNA using the nucleic acid-loaded pulmonary surfactant-based particles prepared in Example 2, the following experiment was performed. Specifically, the following experiment was performed to compare the effects of negative control siRNA and luciferase siRNA itself, to confirm the effect difference between when luciferase siRNA was delivered through a complex and when delivered in the form of pulmonary surfactant particles, and to confirm the difference in efficiency between lipofectamine 3000 and the pulmonary surfactant in a cell culture environment.

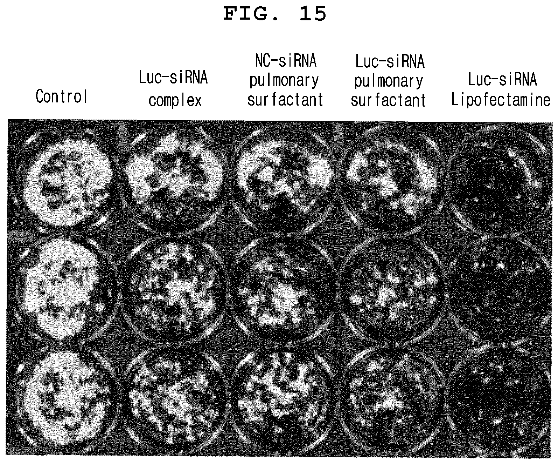

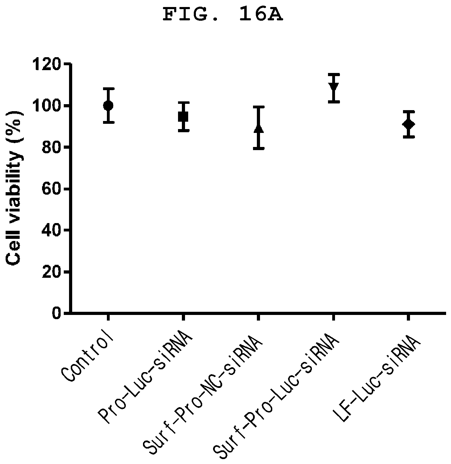

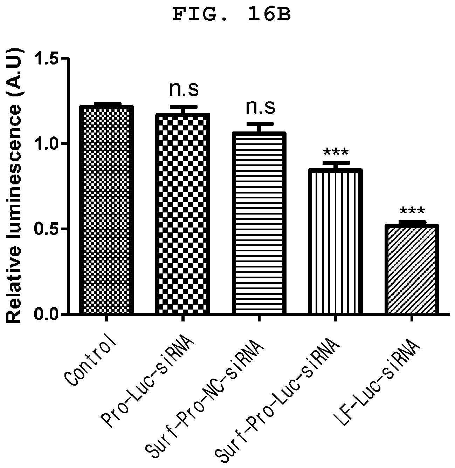

First, A549-luciferase cells were cultured in a 24-well plate (30000 cells/well), and the cells were treated with a negative control, protamine-luciferase siRNA complex (Pro-Luc-siRNA), pulmonary surfactant-protamine-negative control siRNA (Surf-Pro-NC-siRNA), pulmonary surfactant-protamine-luciferase siRNA (Surf-Pro-Luc-siRNA), and lipofectamine 3000-luciferase siRNA (LF-Luc-siRNA). At this time, each group was treated with siRNA at a concentration of about 200 pmol/mL. The cells were cultured for 24 hours, and after washing the cell medium, the cells were further cultured for 24 hours to decrease protein expression. Then, 10 µL of luciferin (12.5 mg/mL) was treated to each well to confirm luminescence, and luminescence was measured using IVIS equipment. In addition, MTT assay was performed to confirm the cell death rate. The results are shown in Figures 15 and 16.

As a result, the protein expression reduction ability could not be confirmed with the protamine-luciferase siRNA complex alone, and when luciferase siRNA was delivered in the form of the pulmonary surfactant particles, the protein expression reduction ability could be confirmed. When compared with the control group, there was a statistically significant difference, but the efficiency was lower than that of lipofectamine. However, unlike the pulmonary surfactant, lipofectamine has a disadvantage that it cannot be used in vivo at all, so the pulmonary surfactant particles could be effectively used for in vivo transfection in the lungs.

### Experimental Example 8: Confirmation of lung cancer apoptosis using pulmonary surfactant

First, A549-luciferase cells were cultured in a 24-well plate (30000 cells/well), and the cells were treated with a negative control, protamine-luciferase siRNA complex (Pro-Luc-siRNA), pulmonary surfactant-protamine-negative control siRNA (Surf-Pro-NC-siRNA), pulmonary surfactant-protamine-luciferase siRNA (Surf-Pro-Luc-siRNA), and lipofectamine 3000-luciferase siRNA (LF-Luc-siRNA). At this time, each group was treated with siRNA at a concentration of about 200 pmol/mL. The cells were cultured for 24 hours, and after washing the cell medium, the cells were further cultured for 24 hours to decrease protein expression. Then, 10 µL of luciferin (12.5 mg/mL) was treated to each well to confirm luminescence, and luminescence was measured using IVIS equipment. In addition, MTT assay was performed to confirm the cell death rate. The results are shown in Figures 15 and 16.

As a result, the protein expression reduction ability could not be confirmed with the protamine-luciferase siRNA complex alone, and when luciferase siRNA was delivered in the form of the pulmonary surfactant particles, the protein expression reduction ability could be confirmed. When compared with the control group, there was a statistically significant difference, but the efficiency was lower than that of lipofectamine. However, unlike the pulmonary surfactant, lipofectamine has a disadvantage that it cannot be used in vivo at all, so the pulmonary surfactant particles could be effectively used for in vivo transfection in the lungs.

### KRAS silencing siRNA (Human)

- Sense: 5'-GUCUCUUGGAUAUUCUCGA-3' (SEQ. ID. NO: 1)
- Antisense: 3'-UCGAGAAUAUCCAAGAGAC-5' (SEQ. ID. NO: 2)

### RPN2 silencing siRNA (Human ribophorin II)

- Sense: 5'-GGCCACUGUUAAACUAGAACA-3' (SEQ. ID. NO: 3)
- Antisense: 3'-GUCCGGUGACAAUUUGAUCUU-5' (SEQ. ID. NO: 4)

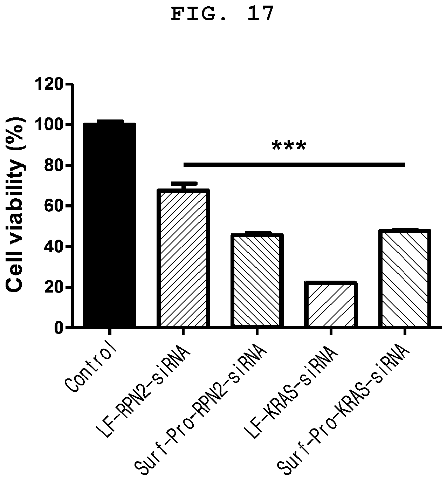

A549-luciferase cells were cultured in a 24-well plate (30000 cells/well), and the cells were treated with a negative control, lipofectamine 3000-RPN2 siRNA (LF-PRN2-siRNA), pulmonary surfactant-protamine-RPN2 siRNA (Surf-Pro-RPN2-siRNA), lipofectamine 3000-KRAS siRNA, and pulmonary surfactant-protamine-KRAS siRNA (Surf-Pro-KRAS-siRNA). At this time, each group was treated with siRNA at a concentration of about 300 pmol/mL. The cells were cultured for 24 hours, and after washing the cell medium, the cells were further cultured for 24 hours to decrease protein expression. Then, MTT assay was performed to confirm the cell death rate. The results are shown in Figure 17.

As shown in Figure 17, when both KRAS-siRNA and RPN2-siRNA were delivered to cells in the form of pulmonary surfactant particles, they showed excellent apoptotic ability to type II alveolar cell-derived lung cancer. The group treated with lipofectamine for comparison also showed similar results. Therefore, it was confirmed that the siRNA-loaded pulmonary surfactant particles of Examples according to the present invention were excellent in suppressing the protein expression in the lungs delivered by inhalation.

Therefore, the pulmonary surfactant particles according to the present invention can be used to effectively deliver nucleic acids such as siRNA into lung cells as a means for suppressing the abnormal protein expression induced in lung diseases. Lung cancer, a representative lung disease, is caused by various oncogenes and tends to get worse as the corresponding proteins are overexpressed. Therefore, a technology capable of inhibiting oncogenes is required to suppress lung cancer, and the inhibition can be achieved using the nucleic acid-loaded pulmonary surfactant-based particles according to the present invention.

### Example 3: Preparation of protein-loaded pulmonary surfactant-based particles-2

Through the following experimental process, the immunoprotein GM-CSF-loaded pulmonary surfactant-based particles were prepared.

The specific experimental procedure is as follows.
(1) A pulmonary surfactant solution was prepared by dissolving pulmonary surfactant powders (Newfacten) at a concentration of 10 mg/ml in a mixed solution of chloroform and methanol (2:1, v:v).
(2) A GM-SCF solution (1 ml) was prepared by dissolving GM-SCF in distilled water at a concentration of 10 µg/ml.
(3) A protamine sulfate solution of 10 times the mole of GM-SCF was prepared.
(4) A lipid film was formed using the pulmonary surfactant (1 mg) prepared in (1) above.
(5) 1 ml of the GM-SCF solution prepared in (2) and the protamine sulfate solution prepared in (3) were added to the lipid film and mixed on a hot plate at a temperature of about 45°C to hydrate the pulmonary surfactant lipid film. The particles produced during the hydration process were made to have an average diameter of 400 nm using an extruder kit (nanoparticulation). In this process, the protamine protein and GM-SCF were bound to the pulmonary surfactant-based particles.
(6) To remove the protein not loaded in the particles, dialysis was performed for at least 8 hours with a 100 kDa membrane in distilled water.
(7) The size and charge of the prepared particles were measured using DLS equipment, and the amount of GM-SCF loaded in the prepared particles was measured using an ELISA kit.

The results are shown in Table 4 below.

**[Table 4]**

| | Size (nm) | Surface charge (mV) | Amount of loaded GM-CSF (µg) |
|---|---|---|---|
| GM-CSF | - | -9.4 | - |
| GM-CSF-loaded pulmonary surfactant particles | 317.5 | -17.5 | 1.06 |

10 µg of GM-CSF, a negatively charged immunoprotein, was loaded in 1 mg of the pulmonary surfactant using electric charge. As a result, it was confirmed that the pulmonary surfactant particles loaded with GM-CSF had a size of about 300 nm and a charge of about -20 mV. When the amount of loaded GM-CSF was measured after removing the unloaded protein, it was confirmed that about 1.06 µg of the protein was loaded. Through this, it was confirmed that GM-CSF, an immunoprotein, could be efficiently loaded in the pulmonary surfactant-based particles using protamine.

## Claims

1. A complex for delivery of a protein or nucleic acid,
wherein the complex is a protein or nucleic acid bound to a liposome prepared with a pulmonary surfactant.

2. The complex according to claim 1, wherein the protein or nucleic acid bound to the liposome is coupled to the liposome via a positively charged protein or peptide.

3. The complex according to claim 2, wherein the positively charged protein is protamine.

4. The complex according to claim 1, wherein the protein bound to the liposome is a negatively charged protein.

5. The complex according to claim 1, wherein the complex is for selectively delivering a liposome-bound protein or nucleic acid to the lung.

6. The complex according to claim 1, wherein the pulmonary surfactant is a lipoprotein complex produced in type II alveolar cells.

7. The complex according to claim 1, wherein the pulmonary surfactant contains a membrane protein.

8. The complex according to claim 1, wherein the complex targets type II alveolar cell-derived lung cancer cells.

9. The complex according to claim 1, wherein the complex has an average diameter in the range of 250 to 1200 nm.

10. A method for preparing a complex for delivery of a protein or nucleic acid comprising the following steps:
preparing a first solution in which a pulmonary surfactant is dissolved;
forming a lipid film by drying the prepared solution in which the pulmonary surfactant is dissolved; and
preparing a complex in which a binding target protein or nucleic acid is bound to a liposome prepared with a pulmonary surfactant by treating the formed lipid film with a second solution in which the binding target protein or nucleic acid is dissolved to hydrate.

11. The method for preparing the complex according to claim 10, wherein in the step of hydrating the formed lipid film by treating the second solution in which the binding target protein or nucleic acid is dissolved, the third solution in which the positively charged protein or peptide is dissolved is also treated to allow the protein or nucleic acid to bind to the liposome through the positively charged protein or peptide.

12. The method for preparing the complex according to claim 11, wherein when performing the method for preparing the complex, 100 to 140 weight part of the pulmonary surfactant is used based on 1 weight part of the binding target protein or nucleic acid and at the same time, 1 to 10 mol of the positively charged protein or peptide is used based on 1 mol of the binding target protein or nucleic acid.

13. The method for preparing the complex according to claim 11, wherein when performing the method for preparing the complex, 100 weight part of the pulmonary surfactant is used based on 1 weight part of the binding target protein or nucleic acid and at the same time, 10 mol of the positively charged protein or peptide is used based on 1 mol of the binding target protein or nucleic acid.

14. The method for preparing the complex according to claim 11, wherein when performing the method for preparing the complex, 0.5 to 20 mol of the positively charged protein or peptide is used based on 1 mol of the binding target protein or nucleic acid.

15. The method for preparing the complex according to claim 10, wherein the hydration is carried out at a temperature of 30 to 90°C.

16. The method for preparing the complex according to claim 10, wherein a step of adjusting the diameter of the complex is further included after performing the hydration step.
